# EUROPEAN PATENT SPECIFICATION

(11) **EP 1 103 264 B1**
(45) Date of publication and mention of the grant of the patent: **30.05.2007**
(21) Application number: 00124968.9
(22) Date of filing: 16.11.2000
(51) Int. Cl.: A61K 33/00, A61K 33/40, A61P 17/02, C25B 1/10, C02F 1/461

(54) **Active oxygen containing solution for promoting growth of tissue cells at wound sites**
Aktivsauerstoff enthaltende Lösung zur Förderung von Gewebewachstum an Wundstellen
Solution contenant de l'oxygène actif favorisant la croissance tissulaire aux sites des lésions

(30) Priority: 17.11.1999 JP 32699399
(43) Date of publication of application: 30.05.2001
(73) Proprietor: OCULUS INNOVATIVE SCIENCES, INC., Petaluma, California 94954 (US)
(72) Inventor: Yahagi, Naoki, Gamou-gun, Shiga (JP); Sumita, Osao, c/o Coherent Technology Co.,Ltd., Tokyo (JP)
(74) Representative: Hoeger, Stellrecht & Partner Patentanwälte

(56) References cited:
- EP-A- 0 368 812
- EP-A- 0 601 891
- EP-A- 0 949 205
- US-A- 4 296 103
- US-A- 5 906 810
- DATABASE WPI Section Ch, Week 200009 Derwent Publications Ltd., London, GB; Class D15, AN 2000-098450 XP002253100 & CN 1 231 994 A (WANG S), 20 October 1999 (1999-10-20)

## Description

### BACKGROUND OF THE INVENTION

### Field of the Invention

This invention concerns the making of a solution for promoting the growth of tissue cells at the site of a wound, a process that promotes regeneration of tissue and wound healing.

### Related Art Statement

"Wound" means a pathological state in which tissue (either inside or on the outer surface of a biobody) is fragmented or damaged, with accompanying less or restriction of the functions of the affected tissues.

It has long been known that when a wound is inflicted, the affected region heals through four steps: "blood vessel reaction", "blood clotting", "inflammation", and "reconstruction of tissue".

Specifically, when a biobody's tissues are damaged so as to cause bleeding, the blood vessel reactions that occur lead first to the formation of clots together with constriction of blood vessels, and this is followed by dilation of the blood vessels after the bleeding stops.

This dilation increases the blood flow at the periphery of the wound, and blood cells and plasma are transferred to, and coagulated at the wound site to cause further clotting, which temporarily closes the wound.

Further, immunoprotective cells gather at the periphery of a wound when the blood flow has increased; to activation of cell-mediated or humoral immunoprotective reactions which cause inflammation.

Finally, the tissues of blood vessels themselves are reconstructed by the action of transmission factors or growth factors released within the wound region, and growth factors secreted by platelets or macrophages provide stimulation to increase the movement of fibroblasts so as to promote reconstruction of connective tissues and, further, form granulations.

In such situation, it is now known that active oxygen produced from bioprotective cells (such as neutrophils or macrophages) that have gathered at the wound site act as biosignals and activate various enzymes and factors to promote the reconstruction of the tissues. That is, a necessary condition for tissue reconstruction is that the relevant enzymes or factors are activated, for which it is necessary that active oxygen is produced in the region of the wound.

It should be mentioned here that drugs used for promoting wound healing are generally hemostatic (for suppressing bleeding in the wound region), anti-inflammatory (for suppressing inflammation), sterilizers (for sterilization so as to prevent miscellaneous bacteria from invading the wound), or drugs having more than one of the above pharmaceutical effects. However, at present "reconstruction of tissue" (the final stage of wound healing has to rely on the autotherapy inherent in living bodies.

EP-A-0 601 891 describes an antifungal and skin healing promoting agent comprising a hypochlorite, a sulfite, a nitrite, a chlorate, hydrogen peroxide, ozone water, a nitrate, and water.

US-A-5 906 810 describes a composition for prophylaxis and treatment of oral lesions comprising water, a peroxide, and a bicarbonate.

US-A-4 296 103 describes an aqueous solution with therapeutic value comprising boron-stabilized chlorine oxides.

CN 1 231 994 A describes (in its abstract) a household ozone and oxygen enriched water generator, yielding high-concentration ozone and oxygen enriched water that has health-care function.

In view of the above, it is the technical object of this invention to manufacture a chemical solution for promoting the growth of tissue cells at wound sites, specifically to aid wound healing by promoting the reconstruction of tissues by enhancing certain biochemical reactions in and around the region of the wound.

This object is achieved by a solution according to claim 1.

Particular embodiments of such solutions are defined in claims 2 to 10.

Claim 11 relates to a method of promoting tissue cell growth in an animal by administering electrolyzed water containing active oxygen and halogen ions as prime ingredients, wherein the active oxygen contains singlet oxygen, superoxide, and hydroxy radical.

A particular embodiment of such a method is defined in claim 12.

The present invention allows the aforementioned object to be achieved by manufacturing an aqueous solution for the promotion of the growth of the tissue cells at wound. Particular embodiments of this solution have nine important features, which are listed below.
(1) The manufactured solution comprises its prime ingredient. The active oxygen can include (i) singlet oxygen (¹O₂) formed by excitation of triplet oxygen, (ii) superoxide (O₂^{.-}) formed by reduction of oxygen by a single electron, and (iii) hydroxy radical (HO), as well as peroxy radical (ROO-), alkoxy radical (RO), and hydroperoxide (ROOH) formed by reactions with biobody ingredients such as unsaturated aliphatic acids (R).
   When the solution for promoting the growth of tissue cells manufactured using this invention is applied to a wound region, the active oxygen by supplied from outside the biobody supplements the active oxygen produced by bioprotective cells (such as neutrophils or macrophages) gathered at the wound. As a result, the concentration of active oxygen at the wound region is increased, producing a state equivalent to one in which a large quantity of biosignals secreted by the tissues themselves. The consequence of this is that reconstruction of tissue cells is promoted.
(2) The manufactured solution comprises water containing not only active oxygen [see (1) above], but also halogen ions as a prime ingredient. On the basis of experiments conducted by the present applicants, it is considered that the solution is more similar to the body's own fluids when halogen ions are present, since halogen ions are a constituent of the fluid formed by tissue cells, and since it and the active oxygen contained in this solution function effectively as biosignals.
(3) The active oxygen present in the manufactured solution is formed by the electrolysis of water. Consequently, it can be produced quite simply, and at minimal cost, by hospitals themselves once electrolysis apparatus is available there.
(4) The manufactured a solution contains, as a prime ingredient, an anode-electrolyzed water obtained by the use of an electrolysis vessel partitioned by a diaphragm into an anode chamber and a cathode chamber, and by supplying water to one of these chambers, and water containing dissolved halogen ions to the other.
(5) The anode-electrolyzed water mentioned in (4) can be obtained by the use of a two-chamber electrolysis vessel in which (i) the diaphragm is formed of a fluoric cation-exchange membrane, and (ii) the anode electrode, or both the anode electrode and the cathode electrode, is in intimate contact with the cationic-exchange membrane.
(6) The anode-electrolyzed water mentioned in (4) and (5) can also be obtained by the use of a three-chamber electrolysis vessel in which an intermediate chamber is located between, and separated by a pair of diaphragms from an anode chamber and a cathode chamber. In this vessel, water is supplied to both the anode chamber and the cathode chamber, and water containing dissolved halogen ions is supplied to the intermediate chamber. Water containing both active oxygen and halogen ions can be formed efficiently using the two-chamber apparatuses described in (4) and (5) or the three-chamber apparatus described in (6).
(7) The manufactured solution contains chlorine as the halogen ion.
(8) The manufactured solution has its pH adjusted to between 6 and 8, which is similar to the pH (7.4) of body fluids.
(9) Sodium chloride (NaCl) is added to the manufactured solution until solution is isotonic with body fluids (i.e., of a similar osmotic pressure). The manufactured solution has ingredients in common with the body fluids, as well as a similar pH and osmotic pressure, and it is considered that as a consequence, the active oxygen contained therein functions more effectively as a biosignal. Furthermore, it has been experimentally confirmed that a solution manufactured using our invention that is at a pH similar to that of the body fluids is effective for internal wounds as well as for wounds at the body's external surface.

### DESCRIPTION OF THE ACCOMPANYING DRAWINGS

Fig. 1 is an explanatory view illustrating a two-chamber electrolysis apparatus that can be used to manufacture a solution for promoting the growth of tissue cells at wound sites.
Fig. 2 is an explanatory view illustrating a second type of two-chambers electrolysis apparatus.
Fig. 3 is an explanatory view illustrating the three-chambers type of electrolysis apparatus.
Fig. 4 is a graph showing absorption spectra for anode-electrolyzed water manufactured using our invention, as well as for chlorous acid and hypochlorous acid.
Fig. 5 is a graph showing ESR (electron-spin resonance) spectra for anode- electrolyzed water manufactured using our invention.
Fig. 6 is a table showing effects of various treatments on the recovery of skin wounds with time.
Fig. 7 is a table showing the characteristics of the solutions manufactured using our invention and of other types of water or solution.
Fig. 8 is a table showing effects of various treatments on the recovery of internal wounds with time.

### DESCRIPTION OF PREFERRED EMBODIMENTS

This invention is now explained more fully and concretely by describing its preferred embodiments (with reference to the accompanying drawings).

The invention employs chemicals and the electrolysis of water to form active oxygen for use in a solution for promoting the growth of tissue cells at wound sites. The use of chemicals involves the use of hydrogen peroxide and the form action of OH radicals by Fenton reaction or the use of ozone. Ozone is not itself activated oxygen, but active oxygen is formed in the course of its decomposition.

The active oxygen formed in biobody cells has biosignaling functions. Hence, for active oxygen supplied from the outside to a wound region to function as an effective agent for the promotion of the growth of tissue cells, it is desirable that the solution within which it is contained should approximate as closely as possible to the body fluids composition, pH, and osmotic pressure.

In the solution manufactured as described in this application, halogen ions (typically represented by chlorine ions) and oxygen series (represented by an oxidative substance such as ozone) are used in combination.

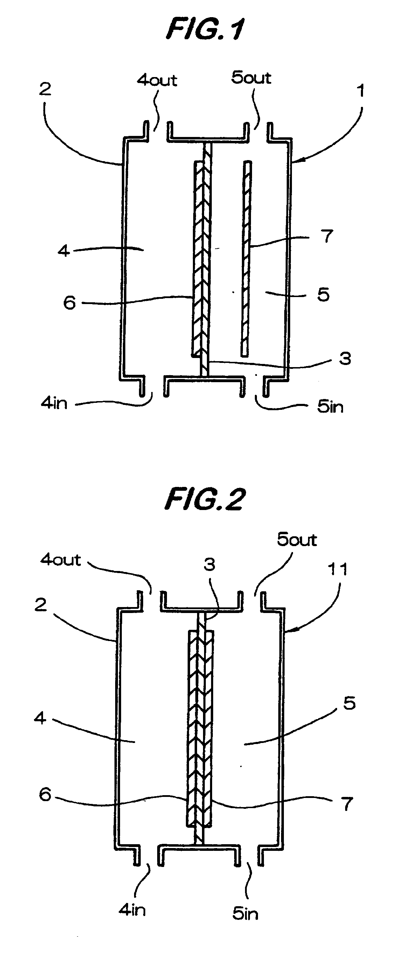

The electrolysis apparatuses 1,11, and 12 (see Figs 1, 2, and 3) can each form such a mixture easily. In Figs 1, 2, and 3, the same or similar parts carry the same reference number in each figure. A detailed explanation of the various parts will be given lates.

In the two-chamber electrolysis apparatus shown in Fig. 1, an electrolysis vessel 2 is partitioned by a fluoric cation exchange membrane 3, acting as a diaphragm, into an anode chamber 4 and a cathode chamber 5. Anode electrode 6 is in intimate contact with the cationic exchange membrane 3, while cathode electrode 7 is located within the cathode chamber 5. In the anode chamber 4 and the cathode chamber 5, are provided inlets 4in and Sin, respectively, and outlets 4out and 5out, respectively. Platinum-plated titanium is used for each of the electrodes 6 and 7. Purified water is supplied to the anode chamber 4 and saline formed by dissolving sodium chloride in purified water is supplied to the cathode chamber 5.

In the second type of two-chamber electrolysis apparatus (11, shown in Fig. 2), both the anode electrode 6 and the cathode electrode 7 are brought into intimate contact with cation exchange membrane 3.

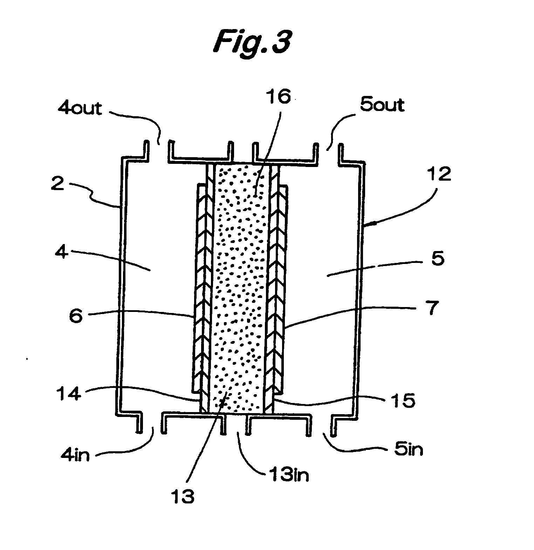

A three-chamber type electrolysis vessel (shown as 2 in Fig. 3), may also be used; in this, an intermediate chamber 13 is located between anode chamber 4 and cathode chamber 5. Chambers 4, 5, and 13 in the three-chamber model are partitioned by means of a pair of diaphragms 14 and 15. In each of the diaphragms 14 and 15, Naphion 117 (trade name of product manufactured by DuPont Co.), acting as a fluoric cation-exchange membrane, and AMV (trade name of product manufactured by Tokuyama Soda Co.), acting as an anion-exchange membrane were used together and an anode electrode 6 and a cathode electrode 7 are placed in intimate contact with these diaphragms, as shown in Fig.3. Further, a granular cation-exchange resin 16 fills the intermediate chamber 13.

When the three-chamber electrolysis apparatus 12 is used, saline is supplied to intermediate chamber 13 via inlet 13in, while purified water is supplied to anode chamber 4 and cathode chamber 5 via inlets 4in and 5in, respectively.

When electrolysis is conducted using electrolysis apparatus 1, 11, or 12 (Figs. 1, 2, and 3, respectively) sufficient chlorine ions are not supplied to the surface of anode electrode 6, so an oxidative decomposing reaction occurs as shown below.

2H₂O + O₂ - 4e⁻ → 4H⁺ + 2O₂

2H₂O - 3e- → 3H⁺ + HO₂

2H₂O - 2e⁻ → 2H⁺ + H₂O₂

As described above, the anode-electrolyzed water formed in the anode chamber contains ozone, active oxygen, and hydrogen peroxide.

Further, since a proportion of the chlorine ions present moves from cathode chamber 5 or intermediate chamber 13 into anode chamber 14, oxygen series oxidative substances and chlorine ions are necessarily present together.

In an aqueous solution in which oxygen series oxidative substances and halogen ions are present together, complex compounds are formed transiently between the various substances. For instance, when ozone and chlorine ions are present together, they do not react instantly to form hypochlorous acid, but rather quasi-stable complexes capable of forming active oxygen are produced.

It is considered that the complex compounds described above have biological effects in the biobody and react with proteins or amino acids to form active oxygen. The latter promotes, by biosignaling, the growth of tissue cells.

Since oxidative active species formed from ozone or by electrolysis have inherent antibacterial actions, they function both to promote the growth of tissue cells and to sterilize the wound region during wound healing.

When the three-chamber electrolysis apparatus 12 (shown in Fig. 3) is used, purified water is supplied at a flow rate of 0.5 l/min to anode chamber 4 and also to cathode chamber 5, while saline (aqueous solution of NaCl) is supplied at a flow rate of 0.2 l/min to intermediate chamber 13.

The area of each of the electrodes 6 and 7 is 48 cm² , and the electrolysis current is 5 A.

For the cathode-electrolyzed water the pH is about 11.5 and ORP is -850 mV, while for the anode electrolyzed water the pH is about 2.4 and ORP is -1150 mV.

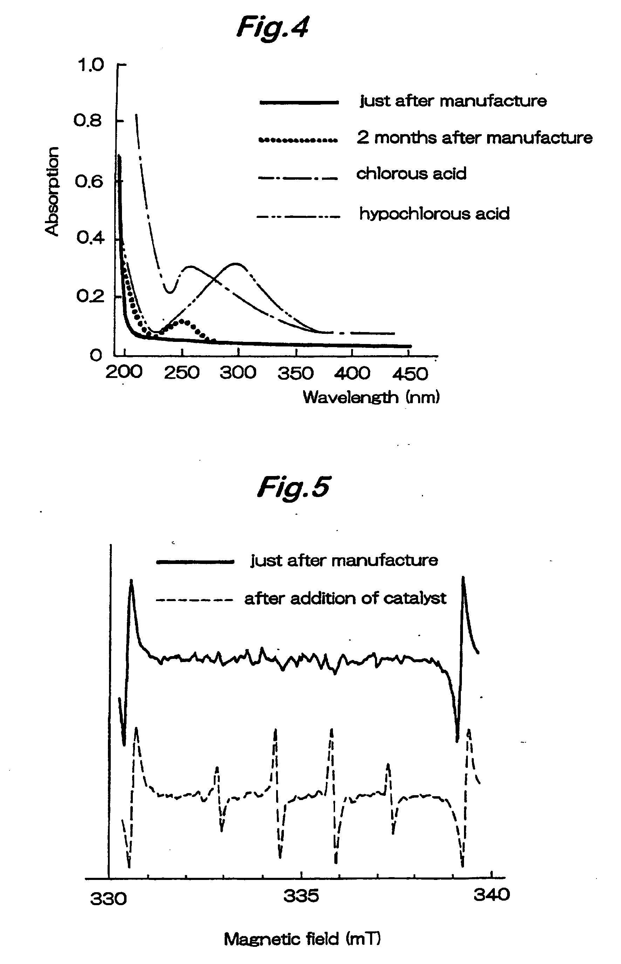

Fig. 4 is a graph showing absorption spectra for the anode-electrolyzed water thus formed (just after formation and after 2 months). According to this graph, since absorption in the near-ultraviolet representing hypochlorite, hypochlorous acid, or chlorous acid is not observed just after formation, it is apparent that hypochlorous acid and chlorous acid are not formed. However, since absorption is observed at a wavelength of 250 nm 2 months after electrolysis, it is apparent that chlorous acid is preset at this time.

Fig. 5 is a graph showing ESR spectra obtained before and after adding DMPO as a radical scavenger to the anode-electrolyzed water thus formed. It can be seen from the graph that although no conspicuous absorption spectra are present for the anode-electrolyzed water before the addition of DMPO, four absorption lines representing OH radicals are present after its addition. Thus, active oxygen is formed in the presence of a catalyst such as iron.

### Experiment 1

The putative tissue cell growth-promoting solution L₁ was adjusted to pH 7.4 by adding an aqueous NaOH (sodium hydroxide) solution to the anode-electrolyzed water, whereas putative tissue cell growth-promoting solution L₂ was left as formed at pH 2.4. For comparison, physiological saline C₁ containing dissolved hypochlorous acid and cathode-electrolyzed water C₂ were used.

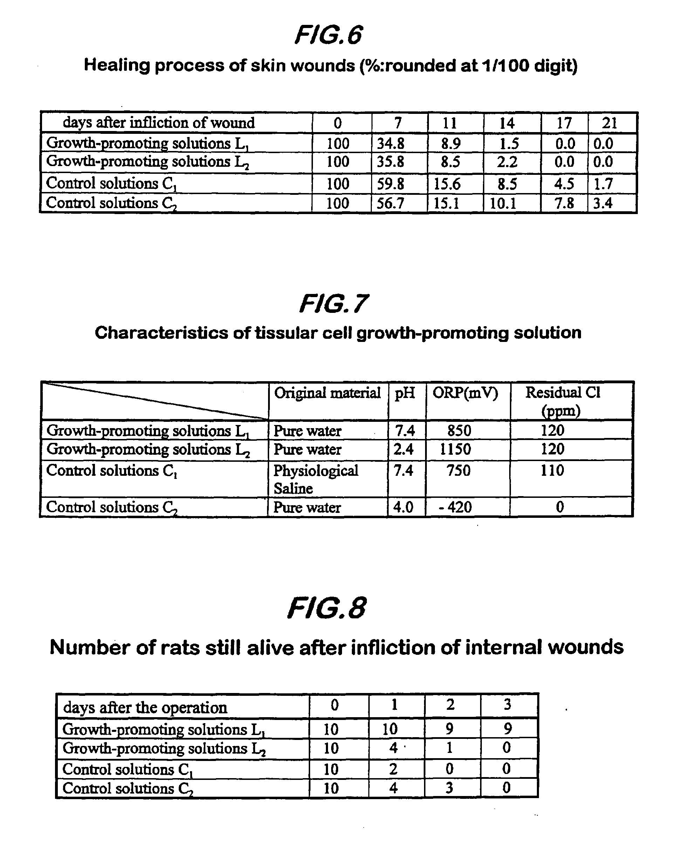

Fig. 6 shows the results of an experiment to examine the recovery with time of skin wounds treated with the above solutions, while Fig. 7 shows the characteristics of the four solutions.

In the experiment, rats were used. The skin on their back was shaved, and then two patches one behind the other, each of 1 cm² and separated by 1cm, were cut out to form wound sites.

Then, but only for the first seven days, putative tissue cell growth-promoting solution (L₁ or L₂), solution C₁, or solution C₂ was applied dropwise twice per day to one wound in each rat (the other being left untreated), care being take that the liquid did not overflow from the wound site. Subsequently, the wound sites were left untouched. The area of the wound sites was determined by a planimetry method, and each is expressed as a percentage of its area on the first day.

The rats were then kept one per cage.

As shown in Fig. 6, rats treated with the tissue cell growth-promoting solutions L₁ or L₂ showed complete healing of the treated wound after 17 days, whereas the rats treated with control solutions C₁ or C₂ did not show complete healing even after 21 days. Untreated wounds were not completely healed even after 21 days.

Further, when NaCl had been added to make the solution isotonic with the body fluids, rats treated with the putative tissue cell growth-promoting solutions (L₁ or L₂) showed more rapidly wounds healing than rats treated with control solutions (C₁ or C₂ ).

### Experiment 2

In this experiment, a peritonitis model was prepared in rats, and the effect examined of the tissue cell growth-promoting solutions. In each rat, the cecum was ligated at a position 3 to 5 mm from the ileum, and then punctured at two places at an intermediate position between the ligation and the distal end of the cecum and near the proximal end of the cecum using an 18 G injection needle. This was done deliberately to cause acute peritonitis.

A catheter was inserted from the back of the rat to pass beneath the skin and into the abdominal cavity. Then, one of the putative tissue cell growth-promoting solutions (L₁ or L₂) or one of the control solutions (C₁ or C₂ ) was injected lcc per 100 g of body weight into the abdominal cavity so that it bathed the region expected to be afflicted by peritonitis.

The table in Fig. 8 shows the results observed in rats kept in cages for three days after the operation. According to these figures -- which show the number of rats (out of 10 in each group) still alive on each day - the progress to peritonitis was not hindered except in the rats treated with the pH adjusted tissue cell growth-promoting solution L₁. Hence, it is considered that the pH has to be adjusted to neutral to achieve healing of internal wounds.

As described above, when the tissue cell growth-promoting solution manufactured using this invention is applied to a wound region, the active oxygen so supplied (from outside the biobody) supplements active oxygen produced from the body's bioprotective cells (such as neutrophils or macrophages) gathered at the site of the wound. As a consequence, the concentration of active oxygen at the wound site is increased, mimicking a state in which a large quantity of biosignals are secreted by the biobody itself. In this way, a solution manufactured using our invention can provide an excellent enhancement of the reconstruction of tissue cells that would occur naturally. As a result, wound healing takes place much more quickly.

## Claims

1. A solution for promoting the growth of tissue cells at the site of a wound, comprising water containing active oxygen and halogen ions as prime ingredients, wherein the active oxygen contains singlet oxygen, superoxide, and hydroxy radical.

2. A solution as defined in claim 1 for promoting the growth of tissue cells at the site of a wound, wherein the halogen ions are chlorine ions.

3. A solution as defined in claim 1 or 2 for promoting the growth of tissue cells at the site of a wound in which the active oxygen is formed by the electrolysis of water.

4. A solution as defined in claim 1 for promoting the growth of tissue cells at the site of a wound, wherein the active oxygen contains singlet oxygen, superoxide, and hydroxy radical manufactured using an electrolysis vessel partitioned by a diaphragm into an anode chamber and a cathode chamber, and by supplying water to one of these chambers and supplying water containing dissolved halogen ions to the other.

5. A solution as defined in claim 4 for promoting the growth of tissue cells at the site of a wound, wherein the active oxygen contains singlet oxygen, superoxide, and hydroxy radical manufactured using a two-chamber electrolysis vessel in which the diaphragm is a fluoric cation-exchange membrane and the anode electrode is in intimate contact with this cationic-exchange membrane.

6. A solution as defined in claim 1 for promoting the growth of tissue cells at the site of a wound, wherein the active oxygen contains singlet oxygen, superoxide, and hydroxy radical manufactured using three-chamber electrolysis vessel in which an intermediate chamber is located between an anode chamber and a cathode chamber, and separated from them by a pair of diaphragms, and supplying water to both the anode chamber and the cathode chamber and supplying water containing dissolved halogen ions to the intermediate chamber.

7. A solution as defined in claim 4, 5, or 6 for promoting the growth of tissue cells at the site of a wound wherein the halogen ions are chlorine ions.

8. A solution as defined in any one of claims 1 to 7 for promoting the growth of tissue cells at the site of a wound wherein the pH is between 6 and 8.

9. A solution as defined in any one of claims 1 to 8 for promoting the growth of tissue cells at the site of a wound wherein sodium chloride is added to make the solution isotonic with body fluids.

10. A solution as defined in claim 8 for promoting the growth of tissue cells at the site of a wound, wherein the solution contains anode-electrolyzed water derived from pure water as an original material, said solution being adjusted to a pH of 7.4 by adding an aequous sodium hydroxide solution to the anode-electrolyzed water and said solution having a positive oxidative-reductive potential of +850 millivolts (mV) and a residual Cl content of 120 ppm.

11. A method of promoting tissue cell growth in an animal by administering electrolyzed water containing active oxygen and halogen ions as prime ingredients, wherein the active oxygen contains singlet oxygen, superoxide, and hydroxy radical.

12. A method as defined in claim 11, wherein the water has a pH of between 6 and 8.

## Patentansprüche

1. Lösung zum Fördern des Wachstums von Gewebezellen an dem Ort einer Wunde, umfassend Wasser, welches Aktivsauerstoff und Halogenionen als Hauptbestandteile enthält, wobei der Aktivsauerstoff Singulettsauerstoff, Superoxid und ein Hydroxyradikal enthält.

2. Lösung nach Anspruch 1 zum Fördern des Wachstums von Gewebezellen an dem Ort einer Wunde, wobei die Halogenionen Chlorionen sind.

3. Lösung nach Anspruch 1 oder 2 zum Fördern des Wachstums von Gewebezellen an dem Ort einer Wunde, wobei der Aktivsauerstoff durch die Elektrolyse von Wasser gebildet ist.

4. Lösung nach Anspruch 1 zum Fördern des Wachstums von Gewebezellen an dem Ort einer Wunde, wobei der Aktivsauerstoff Singulettsauerstoff, Superoxid und ein Hydroxyradikal enthält, hergestellt mittels eines Elektrolysebehälters, der durch ein Diaphragma in eine Anodenkammer und eine Kathodenkammer getrennt ist, und durch Zuführung von Wasser zu einer dieser Kammern und Zuführung von gelöste Halogenionen enthaltendem Wasser zu der anderen.

5. Lösung nach Anspruch 4 zum Fördern des Wachstums von Gewebezellen an dem Ort einer Wunde, wobei der Aktivsauerstoff Singulettsauerstoff, Superoxid und ein Hydroxyradikal enthält, hergestellt mittels eines Zweikammer-Elektrolysebehälters, wobei das Diaphragma eine Fluor-Kationenaustauschmembran ist und wobei die Anodenelektrode in innigem Kontakt mit dieser Kationenaustauschmembran steht.

6. Lösung nach Anspruch 1 zum Fördern des Wachstums von Gewebezellen an dem Ort einer Wunde, wobei der Aktivsauerstoff Singulettsauerstoff, Superoxid und ein Hydroxyradikal enthält, hergestellt mittels eines Dreikammer-Elektrolysebehälters, wobei eine Zwischenkammer zwischen einer Anodenkammer und einer Kathodenkammer angeordnet und von diesen durch ein Paar von Diaphragmen getrennt ist, und Zuführung von Wasser zu sowohl der Anodenkammer als auch der Kathodenkammer und Zuführung von gelöste Halogenionen enthaltendem Wasser zu der Zwischenkammer.

7. Lösung nach Anspruch 4, 5 oder 6 zum Fördern des Wachstums von Gewebezellen an dem Ort einer Wunde, wobei die Halogenionen Chlorionen sind.

8. Lösung nach einem der Ansprüche 1 bis 7 zum Fördern des Wachstums von Gewebezellen an dem Ort einer Wunde, wobei der pH-Wert zwischen 6 und 8 beträgt.

9. Lösung nach einem der Ansprüche 1 bis 8 zum Fördern des Wachstums von Gewebezellen an dem Ort einer Wunde, wobei Natriumchlorid hinzugegeben wird, um die Lösung isotonisch mit Körperfluiden zu machen.

10. Lösung nach Anspruch 8 zum Fördern des Wachstums von Gewebezellen an dem Ort einer Wunde, wobei die Lösung Anoden-elektrolysiertes Wasser enthält, abgeleitet von reinem Wasser als ein Ausgangsmaterial, wobei die Lösung auf einen pH-Wert von 7,4 eingestellt ist durch Zugabe einer wässrigen Natriumhydroxid-Lösung zu dem Anoden-elektrolysierten Wasser und wobei die Lösung ein positives oxidatives-reduktives Potential von +850 Millivolt (mV) und einen Rest-CI-Gehalt von 120 ppm aufweist.

11. Verfahren zum Fördern des Gewebezellenwachstums bei einem Tier durch Verabreichen von elektrolysiertem Wasser, welches Aktivsauerstoff und Halogenionen als Hauptbestandteile enthält, wobei der Aktivsauerstoff Singulettsauerstoff, Superoxid und ein Hydroxyradikal enthält.

12. Verfahren nach Anspruch 11, wobei das Wasser einen pH-Wert aufweist, der zwischen 6 und 8 beträgt.

## Revendications

1. Solution destinée à favoriser la croissance de cellules tissulaires à l'endroit d'une plaie, comprenant de l'eau contenant de l'oxygène actif et des ions halogène en tant qu'ingrédients principaux, dans laquelle l'oxygène actif contient de l'oxygène singulet, un superoxyde et un radical hydroxyle.

2. Solution telle que définie dans la revendication 1 destinée à favoriser la croissance de cellules tissulaires à l'endroit d'une plaie, dans laquelle les ions halogène sont des ions chlorure.

3. Solution telle que définie dans la revendication 1 ou 2 destinée à favoriser la croissance de cellules tissulaires à l'endroit d'une plaie, dans laquelle l'oxygène actif est formé par l'électrolyse d'eau.

4. Solution telle que définie dans la revendication 1 destinée à favoriser la croissance de cellules tissulaires à l'endroit d'une plaie, dans laquelle l'oxygène actif contient de l'oxygène singulet, un superoxyde et un radical hydroxyle, préparée en utilisant un récipient d'électrolyse divisée par un diaphragme en une chambre d'anode et une chambre de cathode, et en introduisant de l'eau dans l'une de ces chambres et en introduisant de l'eau contenant des ions halogène sous forme dissoute dans l'autre.

5. Solution telle que définie dans la revendication 4 destinée à favoriser la croissance de cellules tissulaires à l'endroit d'une plaie, dans laquelle l'oxygène actif contient de l'oxygène singulet, un superoxyde et un radical hydroxyle préparée en utilisant un récipient d'électrolyse à deux chambres dans lequel le diaphragme est une membrane échangeuse de cations fluorure et l'anode est en contact intime avec cette membrane échangeuse de cations.

6. Solution telle que définie dans la revendication 1 destinée à favoriser la croissance de cellules tissulaires à l'endroit d'une plaie, dans laquelle l'oxygène actif contient de l'oxygène singulet, un superoxyde et un radical hydroxyle, préparée en utilisant un récipient d'électrolyse à trois chambres dans lequel la chambre intermédiaire est située entre une chambre d'anode et une chambre de cathode, et séparée de celles-ci par une paire de diaphragmes, et en introduisant de l'eau à la fois dans la chambre d'anode et dans la chambre de cathode, et en introduisant de l'eau contenant des ions halogène sous forme dissoute dans la chambre intermédiaire.

7. Solution telle que définie dans la revendication 4, 5 ou 6 destinée à favoriser la croissance de cellules tissulaires à l'endroit d'une plaie, dans laquelle les ions halogène sont des ions chlorure.

8. Solution telle que définie dans l'une quelconque des revendications 1 à 7 destinée à favoriser la croissance de cellules tissulaires à l'endroit d'une plaie, dans laquelle le pH est compris entre 6 et 8.

9. Solution telle que définie dans l'une quelconque des revendications 1 à 8 destinée à favoriser la croissance de cellules tissulaires à l'endroit d'une plaie, dans laquelle on ajoute du chlorure de sodium pour rendre la solution isotonique par rapport aux fluides corporels.

10. Solution telle que définie dans la revendication 8 destinée à favoriser la croissance de cellules tissulaires à l'endroit d'une plaie, dans laquelle la solution contient, en tant que matière de départ, de l'eau électrolysée sur l'anode provenant d'eau pure, ladite solution étant ajustée à un pH de 7,4 en ajoutant une solution d'hydroxyde de sodium aqueuse à l'eau électrolysée sur l'anode et ladite solution ayant un potentiel oxydo-réducteur positif de +850 millivolts (mV) et une teneur en Cl résiduel de 120 ppm.

11. Procédé destiné à favoriser la croissance de cellules tissulaires chez un animal, dans lequel on administre de l'eau électrolysée contenant de l'oxygène actif et des ions halogène en tant qu'ingrédients amorceurs, l'oxygène actif contenant de l'oxygène singulet, un superoxyde et un radical hydroxyle.

12. Procédé tel que défini dans la revendication 11, dans lequel l'eau a un pH compris entre 6 et 8.
